# EUROPÄISCHE PATENTSCHRIFT

(11) **EP 0 917 869 B1**
(45) Veröffentlichungstag und Bekanntmachung des Hinweises auf die Patenterteilung: **26.01.2005**
(21) Anmeldenummer: 98119422.8
(22) Anmeldetag: 14.10.1998
(51) Int. Cl.: A61K 7/00, A61K 47/32

(54) **Verwendung von W/O-Emulsionen als Verdicker in kosmetischen und pharmazeutischen Zubereitungen**
Use of water-in-oil emulsions as thickening agents in cosmetical and pharmaceutical compositions
Utilisation d'émulsions eau-dans-huile comme épaississants dans des préparations cosmétiques et pharmaceutiques

(30) Priorität: 10.11.1997 DE 19749618
(43) Veröffentlichungstag der Anmeldung: 26.05.1999
(73) Patentinhaber: BASF AKTIENGESELLSCHAFT, 67056 Ludwigshafen (DE)
(72) Erfinder: Tiefensee, Kristin Dr., 67368 Westheim (DE); Schehlmann, Volker Dr., 67354 Römerberg (DE); Rübenacker, Martin Dr., 67122 Altrip (DE)

(56) Entgegenhaltungen:
- EP-A- 0 776 690
- WO-A-95/35089
- DE-C- 3 522 419
- US-A- 5 206 316
- US-A- 5 531 993

## Beschreibung

Die Erfindung betrifft die Verwendung von W/O-Emulsionen, in denen vernetzte, wasserquellbare Polymerisate dispergiert sind, als Verdickungsmittel in kosmetischen oder pharmazeutischen Zubereitungen. Die Erfindung betrifft außerdem W/O-Emulsionen und kosmetische und pharmazeutische Zubereitungen.

In der Formulierung von kosmetischen oder pharmazeutischen Zubereitungen, wie z.B. Hautcremes, -lotionen, Gelen und anderen kosmetischen Pflegeprodukten sowie pharmazeutischen Hautpräparationen werden zur Stabilisierung vernetzte Acrylsäurepolymerisate eingesetzt, die unter dem CTFA-Namen Carbomer bekannt sind. Diese Polymerisate sind Fällungspolymerisate und stellen frei fließende Pulver dar, die nach dem Einrühren in Wasser neutralisiert werden. Dieser Neutralisationsschritt ist notwendig, um die sauren Polymere in die Carboxylate zu überführen, die letztlich für den Viskositätsanstieg und damit Stabilisierung der Formulierung verantwortlich sind. Nachteil dieser pulverförmigen Polymere sind ihre Staubigkeit und die damit verbundene schlechte Benetzbarkeit, die einen längeren Zeitraum zum Homogenisieren bedingt. Geringe Mengen Restlösemittel aus dem Herstellprozeß sind nicht auszuschließen.

Ein nicht staubendes Polymerisat ist in der EP 383 057 beschrieben: Es handelt sich um vernetzte Acrylsäure-Polymere mit nitrilhaltigen Comonomeren, die in einer Wasser/Paraffin-Emulsion hergestellt werden; das Wasser wird azeotrop abdestilliert. Die beschriebenen Emulsionen werden im Textildruck eingesetzt, für die Anwendung in Kosmetikprodukten sind sie nicht geeignet.

In der DE 35 22 419 wird die umgekehrte Emulsionspolymerisation von Acrylamid mit Acrylsäure in einem Verfahrensschritt beschrieben. Der pH-Wert liegt im alkalischen und ist daher für kosmetische Zwecke, vor allem Hautanwendungen ungünstig.

Ein Verfahren zur Herstellung wasserlöslicher oder wasserquellbarer Polymerer in einer W/O-Emulsion beansprucht die EP 126 528, das dadurch gekennzeichnet ist, daß die wasserlöslichen Monomere in Anwesenheit von Emulgatoren unter Zusatz eines speziellen Dispergiersystems bestehend aus Alkanolen polymerisiert werden. Für Kosmetikanwendungen sind die Polymerisate nicht vorgesehen.

Vernetzte Acrylsäurepolymerisate in einer W/O-Emulsion sind außerdem in der EP 297 184 als Wasserabsorbens beschrieben. Der Vernetzungsgrad beträgt max. 0,5 Mol-%; ein Invertierungsmittel ist enthalten. Die Emulgatorkonzentration beträgt mindestens 5 % bezüglich Gesamtdispersion. Die Polymerisation wird öllöslich initiiert. Als Ölkomponente wird z.B. Olivenöl oder Eukalyptusöl verwendet, d.h. Öle, die ungesättigt und damit instabil, oder stark riechend sind.

Aus der DE 42 17 673 A1 sind elektrolytverdickbare Tensidkombinationen bekannt als Basis für kosmetische Zubereitungen. Die hierbei zur Verdickungswirkung notwendigen Elektrolyte und Tenside sind für bestimmte Anwendungen nachteilig.

In der WO 95/35089 werden topisch anwendbare Zusammensetzungen beschrieben, die ein Verdickungsmittel enthalten. Das Verdikkungsmittel ist eine W/O-Emulsion mit einem vernetzten Polymer und einem flüchtigen Öl wie einem Silikon oder einem Isoparaffin als organischer Phase.

Aus der EP 503 853 sind wasserlösliche Verdickungsmittel für kosmetische Formulierungen bekannt, die ein ganz bestimmtes Comonomer (AMPS) enthalten.

Aus der US 5 206 316 sind Lactam-haltige Emulgatoren bekannt, die bei der inversen Emulsionspolymerisation eingesetzt werden.

Aufgabe der vorliegenden Erfindung ist es, Polymerisate bereit zu stellen, die die Nachteile der Pulverprodukte nicht aufweisen und außerdem ein einfaches Formulieren kosmetischer oder dermatologischer Rezepturen ermöglichen: gute Effizienz, leichte Benetzbarkeit, selbstinvertierend, ohne zusätzlichen Neutralisationsschritt einsetzbar und einen pH-Wert von 6 - 7 liefernd. Die Endprodukte sollen ein glattes Aussehen haben und im Falle von Hautpflegeprodukten auf der Haut leicht "brechen".

Die Aufgabenstellung wird gelöst durch die Verwendung von W/O-Emulsionen, in denen vernetzte, wasserquellbare Polymerisate dispergiert sind, wobei die Polymerisate bestehend aus
a) 35 - 100 Gew.-% ionische Monomere, wobei die ionischen Monomere zu 5-80 % neutralisiert sind,
b) 0 - 65 Gew.-% nichtionische Monomere,
c) 0,3 - 1 Mol-% bezogen auf a) und b) mindestens eines mindestens bifunktionellen Monomeren,
wobei die Ölphase aus einem oder mehreren Fettsäureestern besteht, als Verdicker in kosmetischen oder pharmazeutischen Zubereitungen.

Bevorzugt werden W/O-Emulsionen verwendet, deren Ölphase aus Polyglycerinfettsäureestern besteht, insbesondere Fettsäureestern aus einer Polyglycerinmischung, enthaltend Di- und Triglycerin, mit Capryl- und/oder Caprinsäure.

Die Erfindung betrifft weiterhin W/O-Emulsionen, in denen vernetzte, wasserquellbare Polymerisate dispergiert sind,
wobei die Polymerisate bestehend aus
a) 35 - 100 Gew.-% ionische Monomere, wobei die ionischen Monomere zu 5-80 % neutralisiert sind,
b) 0 - 65 Gew.-% nichtionische Monomere,
c) 0,3 - 1 Mol-% bezogen auf a) und b) mindestens eines mindestens bifunktionellen Monomeren, und
wobei die Ölphase aus einem oder mehreren Polyglycerinfettsäureestern aus einer Polyglycerinmischung, die Diglycerin und Triglycerin enthält, mit Fettsäuregemischen, die Capryl- und/oder Caprinsäure enthalten, besteht.

Die Erfindung betrifft weiterhin kosmetische oder pharmazeutische Zubereitungen, die eine W/O-Emulsion enthalten, in der vernetzte, wasserquellbare Polymerisate dispergiert sind, wobei die Polymerisate bestehen aus
a) 35 - 100 Gew.-% ionische Monomere, wobei die ionischen Monomere zu 5-80 % neutralisiert sind,
b) 0 - 65 Gew.-% nichtionische Monomere,
c) 0,3 - 1 Mol-% bezogen auf a) und b) mindestens eines mindestens bifunktionellen Monomeren, und
wobei die Ölphase aus einem oder mehreren Polyglycerinfettsäureestern besteht.

Der bevorzugte Einsatzzweck für die W/O-Emulsionen ist in hautoder haarkosmetischen Zubereitungen.

Die in der vorliegenden Erfindung beschriebene W/O-Polymerdispersion wird durch umgekehrte Emulsionspolymerisation hergestellt. Im ersten Schritt wird eine wäßrige Monomerlösung mit für diese Systeme typischen W/O-Emulgatoren in einer Ölphase emulgiert. Die Polymerisation wird durch radikalbildende Initiatoren gestartet. Da die Polymerisate als Verdicker fungieren sollen, müssen sie chemisch vernetzt sein. Der Vernetzer wird vorzugsweise der Monomerlösung zugesetzt.

Als Monomere werden bevorzugt ungesättigte C₃-C₅-Carbonsäuren eingesetzt, wie z.B. Acrylsäure, Methacrylsäure, Maleinsäure(anhydrid), Fumarsäure(anhydrid), Itaconsäure oder deren Mischungen. Sie können homopolymerisiert werden oder mit nichtionischen Monomeren copolymerisiert werden. Als solche können ungesättigte Verbindungen Verwendung finden, die sich neben den ionischen Monomeren in Wasser lösen lassen; diese sind z.B. Acrylamid, Methacrylamid, Vinylpyrrolidon, Vinylimidazol, Vinylcaprolactam, Hydroxyalkylester von Carbonsäuren wie z.B. Hydroxyethylacrylsäure. Der Anteil an ionischen Monomeren in der Gesamt-Monomermischung beträgt 35 bis 100 %, vorzugsweise 50 bis 100 %. _{B}esonders bevorzugt ist ein Acrylsäureanteil von > 90 %.

Die ionischen Monomere sind zu 5 bis 80 %, vorzugsweise zu 10 bis 50 %, neutralisiert. Zur Neutralisation können prinzipiell alle Basen verwendet werden, die mit kosmetischen Belangen in Einklang zu bringen sind. Vorzugsweise werden z.B. Triethanolamin, NaOH, Tetrahydroxypropylethylendiamin verwendet. Auch Basengemische sind denkbar.

Die Konzentration der Monomeren in der wäßrigen Lösung vor der Polymerisation beträgt 10 bis 60 %.

Die Vernetzung des Polymerisates erfolgt durch Copolymerisation der Monomeren mit mindestens zweifach ungesättigten wasser- oder öllöslichen Verbindungen. Als solche können fungieren: Methylenbisacrylamid, Divinylpyrrolidon, Allyl(meth)acrylat, Triallylamin, Ethylenglykol-Diarylate (bis 50 EO), (Meth)acrylsäureester von zwei bzw. mehrwertigen Alkoholen wie Trimethylolpropantriacrylat oder Pentaerythrittetraacrylat; die Alkoholfunktionen können hierbei bis zu 50 EO-Gruppen tragen und jeweils unterschiedliche Ethoxylierungsgrade aufweisen. Diese Vernetzer sind zu 0,3 bis 1 Mol-% einzeln oder als Gemisch enthalten. Wasserlösliche Vernetzer werden bevorzugt.

Die Ölphase besteht aus einem oder mehreren Fettsäureester. Diese erfindungsgemäßen Komponenten wirken sich positiv auf die kosmetische Formulierung (Aussehen, Hautgefühl) aus. Solche Komponenten sind z.B. Fettsäureisopropylester wie Isopropylpalmitat, Isopropylmyristat oder Polyglyceride von Fettsäuren, insbesondere Fettsäuregemischen, die mindestens 50 % Capryl- und/oder Caprinsäure enthalten. Bevorzugt sind Polyglycerinfettsäureester auf Basis einer Polyglycerinmischung, die im wesentlichen Mono-, Di-, Tri- und Tetraglycerin, vor allem Di- und Triglycerin, enthält. Der Anteil der Ölphase an der Gesamtemulsion beträgt 15 - 70 %, vorzugsweise 20 - 35 %.

Um die Wasserphase in der organischen Phase zu dispergieren, werden dafür bekannte W/O-Emulgatoren eingesetzt. Der HLB-Wert der verwendeten Emulgatoren liegt zwischen 4 und 8 [HLB-Wert = Hydrophilic/lipophilic balance, vgl. W.C. Giffin, J. Soc. Cosmet. Chem. 1, 311 (1950)]. Solche Emulgatoren sind z.B. Sorbitanmonooleat, Sorbitanmonostearat, Glycerylmonostearat, Blockcopolymere aus Hydroxyfettsäuren-Polyestern und Polyoxyethylen. Sie können alleine oder in Kombination in Gesamtkonzentrationen von 2 bis 10 %, vorzugsweise von 2 bis 5 % bzgl. der Gesamtemulsionen eingesetzt werden.

Es können der Emulsion zusätzlich Emulgatoren mit einem HLB-Wert von über 8 zugesetzt werden in Konzentrationen von 0,25 bis 7 % bzgl. der Gesamtemulsion. Solche Emulgatoren sind z.B. ethoxylierte C₆-C₁₂-Nonylphenole bzw. C₁₂-C₁₈-Fettalkohole; der Ethoxylierungsgrad beträgt 5 bis 20 Mol-%.

Für das Emulgieren der wäßrigen Phase in die Ölphase benötigt man keine speziellen Aggregate, sondern man kann die wäßrige Monomerphase in einem Standardpolymerisationsgefäß durch Rühren mit z.B. einem Ankerrührer emulgieren. Die Drehzahl liegt in Abhängigkeit von der Kesselgeometrie zwischen 30 und 400 UpM.

Als Initiatoren können wasser- und/oder öllösliche Radikalbildner eingesetzt werden wie z.B. Alkali- oder Ammoniumperoxodisulfate, Wasserstoffperoxid, organische Peroxide ggf. mit Redox-Partnern oder Azoinitiatoren. Es können auch Initiatoren unterschiedlicher Zerfallstemperatur gemeinsam oder nacheinander eingesetzt werden. Bezogen auf die Monomermischung verwendet man 0,05 bis 0,5 % Initiator, vorzugsweise 0,05 bis 0,3 %.

Die Temperatur, bei der die Polymerisation erfolgt, liegt zwischen 20 und 150°C; sie kann konstant gehalten werden oder diskontinuierlich verändert werden (z.B. um durch Temperaturerhöhung den Umsatz zu steigern). Man erhält nach der Polymerisation Wasser in Öl-Emulsionen mit einem Feststoffgehalt von 10 bis 40 %, vorzugsweise von 15 bis 35 %. Zur Erhöhung des Feststoffgehaltes können die Emulsionen durch Destillation teilweise oder vollständig entwässert werden.

Die erfindungsgemäßen W/O-Emulsionen vernetzter Polymerisate werden als Verdickungsmittel vorzugsweise in kosmetischen oder pharmazeutischen Anwendungen eingesetzt. Die Polymerisate werden nicht isoliert, sondern in Form der W/O-Emulsion direkt eingesetzt. Typische Einsatzkonzentrationen sind 0,1 bis 0,8 %, vorzugsweise 0,2 bis 0,5 % Wirkstoff (Polymer). Die Verdickungswirkung der W/O-Emulsion tritt direkt nach dem Vermischen der W/O-Emulsion mit der kosmetischen O/W-Emulsion ein; um die optimale Wirkung zu erzielen, ist kein Zusatz eines Invertierungsmittels notwendig. Auch rein wäßrige Systeme lassen sich verdicken. Man erhält ein Cremegel.

### Beispiele

### Allgemeine Vorschrift zur Herstellung der Polymeremulsionen

In einem 2 l Polymerisationsgefäß, das mit Ankerrührer, Thermometer, Stickstoffeinlaß und -auslaß versehen ist, wird jeweils die nachstehende Monomeremulsion vorgelegt. Die polymerisierbare Mischung wird unter Stickstoffstrom 30 min mit der Hälfte der Startermenge emulgiert. Zur Durchführung der Polymerisation wird danach die Temperatur auf 50 bis 55°C angehoben. Nach Zugabe der zweiten Starterhälfte wird die Emulsion 2 h bei 55 bis 65°C nachpolymerisiert. Danach wird auf Raumtemperatur abgekühlt.

In der Tabelle 1 sind die einzelnen Beispiele 1 - 9 mit den jeweils eingesetzten Stoffen angegeben. Die Indizes bezeichnen dabei folgende Stoffe:
¹⁾ Capryl-/Caprinsäure-Triglyceride (Hüls AG, enthaltend 50 - 65 % Capryl und 30 - 45 % Caprinsäure)
²⁾ Sorbitanmonooleat (ICI)
³⁾ ABA-Blockcopolymere aus einem Hydroxystearinsäure-Kondensat und Polyethylenglykol (ICI)
⁴⁾ 2,2'-Azobis(2-amidinopropan)dihydrochlorid (Wako), T (t = 10 h) = 56°C
⁵⁾ 2,2'-Azobis(N,N'-dimethyleneisobutyramidin)dihydrochlorid (Wako), T (t = 10 h) = 44°C

Alle Ansätze enthalten 0,02 % Pentanatriumsalz der Diethylentriaminpentaessigsäure.

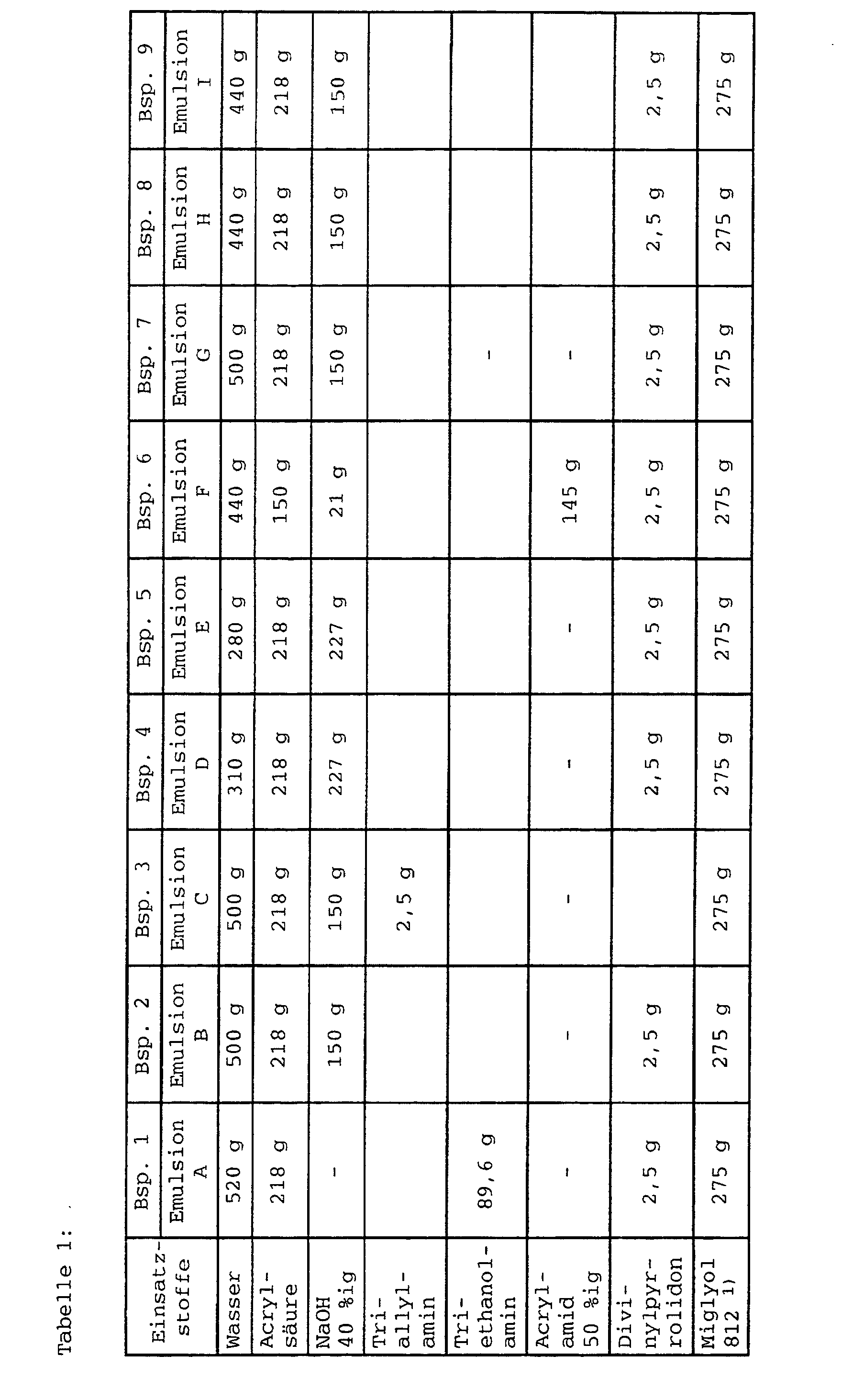

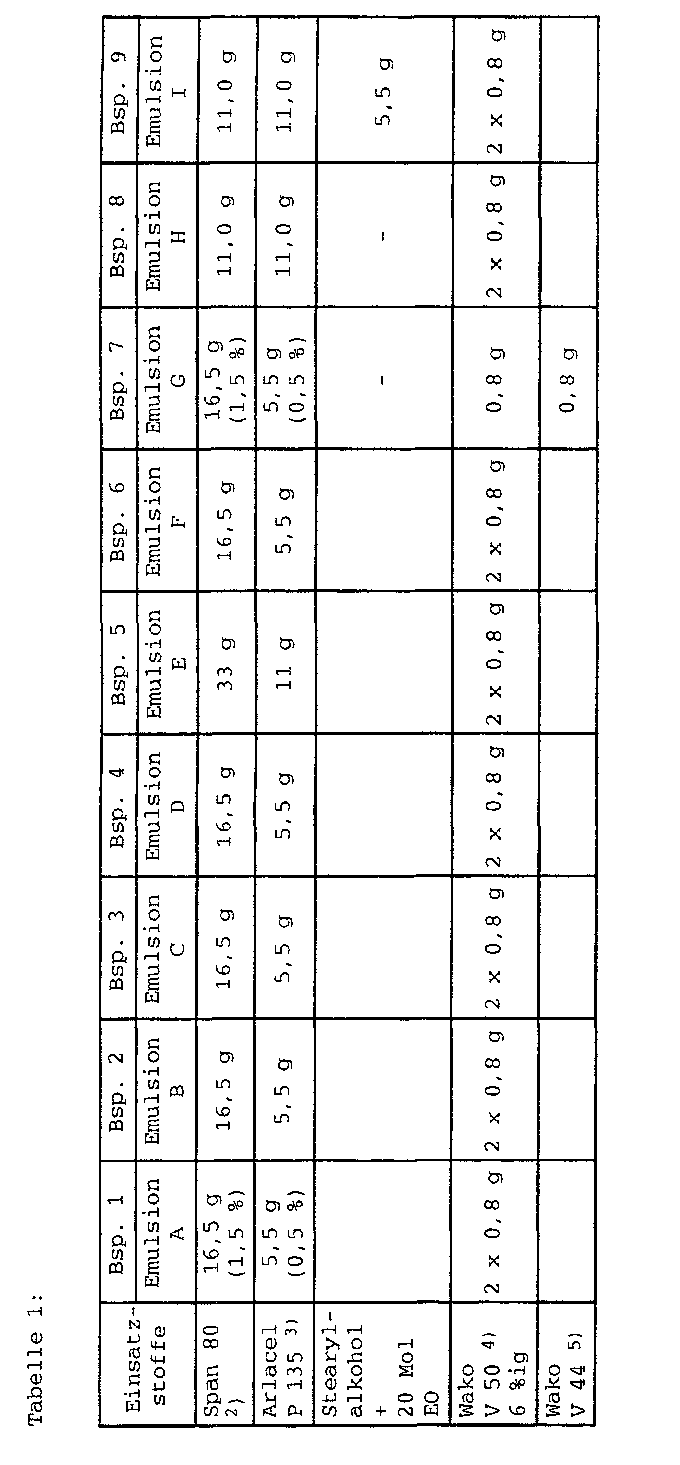

### Vergleichsbeispiele

| Einsatzstoff | Emulsion 1 | Emulsion2 |
|---|---|---|
| Wasser | 280 g | 280 g |
| Acrylsäure | 218 g | 218 g |
| NaOH 40 %ig | 227 g | 227 g |
| Divinylpyrrolidon | 2,5 g | 2,5 g |
| Paraffinöl | 275 g | |
| Abil® 100 ⁴⁾ | | 275 g |
| Sorbitanmonooleat ¹⁾ | 11,0 g | 11,0 g |
| Arlacel® P 135 ²⁾ | 11,0 g | 11,0 g |
| Wako® V 50 ³⁾ 6 %ig | 2 x 0,8 g | 2 x 0,8 g |

| | | |
|---|---|---|
| ¹⁾ Sorbitanmonooleat (ICI) | | |
| ²⁾ ABA-Blockcopolymere aus einem Hydroxystearinsäure-Kondensat und Polyethylenglykol (ICI) | | |
| ³⁾ 2,2'-Azobis(2-amidinopropan)dihydrochlorid (Wako), T (t = 10 h) = 56°C | | |
| ⁴⁾ Dimethylpolysiloxan | | |

### Prüfung der W/O-Emulsionen:

Die Emulsionen wurden in nachfolgenden Rezepturen eingesetzt. Beurteilt wurde die Viskosität (Bookfield RVT) und das Aussehen der Formulierung. Letzteres wurde an einem Ausstrich beurteilt und nach dem Schulnotensystem 1 bis 5 bewertet. "Struktur" gibt Glanz und Oberflächenbeschaffenheit wider, als "Stippen" werden kleine Gelkörper bezeichnet. Sie sind u.a. ein Maß für die Invertierbarkeit der W/O-Emulsion. Struktur: Note 1 bedeutet glatte Oberfläche, glänzend; Note 5 bedeutet narbige Oberfläche, matt. Stippen: Note 1 bedeutet keine Gelkörper wahrnehmbar, Note 5 heißt, es sind deutlich Gelpartikel vorhanden.

### Gel mit etherischen Ölen

| | | |
|---|---|---|
| 1 % | Wirkstoff (Verdickungsmittel) | |
| 49 % | Wasser | |
| 10 % | Ethanol | |
| 7,5 % | Rosmarinöl | |
| 7,5 % | Fichtennadelöl | |
| 2 % | Cremophor® RH 40 | BASF, PEG 40 hydrog. castor. oil |
| 0,1 % | Euxyl® K 100 | Schülke & Mayr, Benzyl-alkohol, Methylchlorisothiazolinon |

Nach dem Solubilisieren der Ölkomponenten wird Wasser und die Verdicker-Emulsion zugegeben und 1 bis 2 min homogenisiert.

| | | | |
|---|---|---|---|
| A | 2 % | Cremophor A6 | BASF, Ceteareth-6 (and) Stearylalkohol |
| | 2 % | Cremophor A 25 | BASF, Ceteareth-25 |
| | 6 % | Mandelöl | |
| | 3 % | Imwitor® 960 K | Hüls, Glyceryl stearat SE |
| | 1,5 % | Lanette® O | Henkel, Cetearylalkohol |
| | 0,5 % | Abil® 100 | Wacker, Dimethicone |
| | 8 % | Luvitol® EHO | BASF, Cetearyl octanoat |
| | | | |
| B | 3 % | 1,2-Propylenglykol | |
| | 2 % | Glycerin | |
| | 3 % | Aloe Vera Gel | |
| | 0,3 % | WIRKSTOFF | |
| | 65,3 % | Wasser | |
| | | | |
| C | 3 % | Collagen CLR | |

Phase A und B werden getrennt auf 80°C erwärmt. Phase B wird in Phase A homogenisiert. Bei 40°C wird Phase C dazugegeben und homogenisiert.

| | | | |
|---|---|---|---|
| A | 0,25 % | Pemulen TR 1 | B.F. Goodrich, Acrylates/C₁₀-C₃₀-Alkyl Acrylate crosspolymer |
| | 7 % | Uvinul® MC 80 | BASF, Octyl Methoxycinnamate |
| | 3 % | Octyl Salicylat | |
| | 6 % | Witconol® APM | Wicto, PPG-3 Myristyl Ether |
| | 1 % | Uvinul® M 40 | BASF, Benzophenon-3 |
| | 0,2 % | WIRKSTOFF | |
| | 2 % | Luvitol® EHO | BASF, Cetearyl octanoat |
| | 0,2 % | (-)-α-Bisabolol | |
| | | | |
| B | 0,1 | Natrosol® 250 HR | Aqualon, Hydroxyethylcellulose |
| | 0,05 % | EDTA BD | BASF Disodium EDTA |
| | 3 % | 1,2-Propylenglykol | |
| | 74,8 % | Wasser | |
| | | | |
| C | 0,2 % | Triethanolamin | |

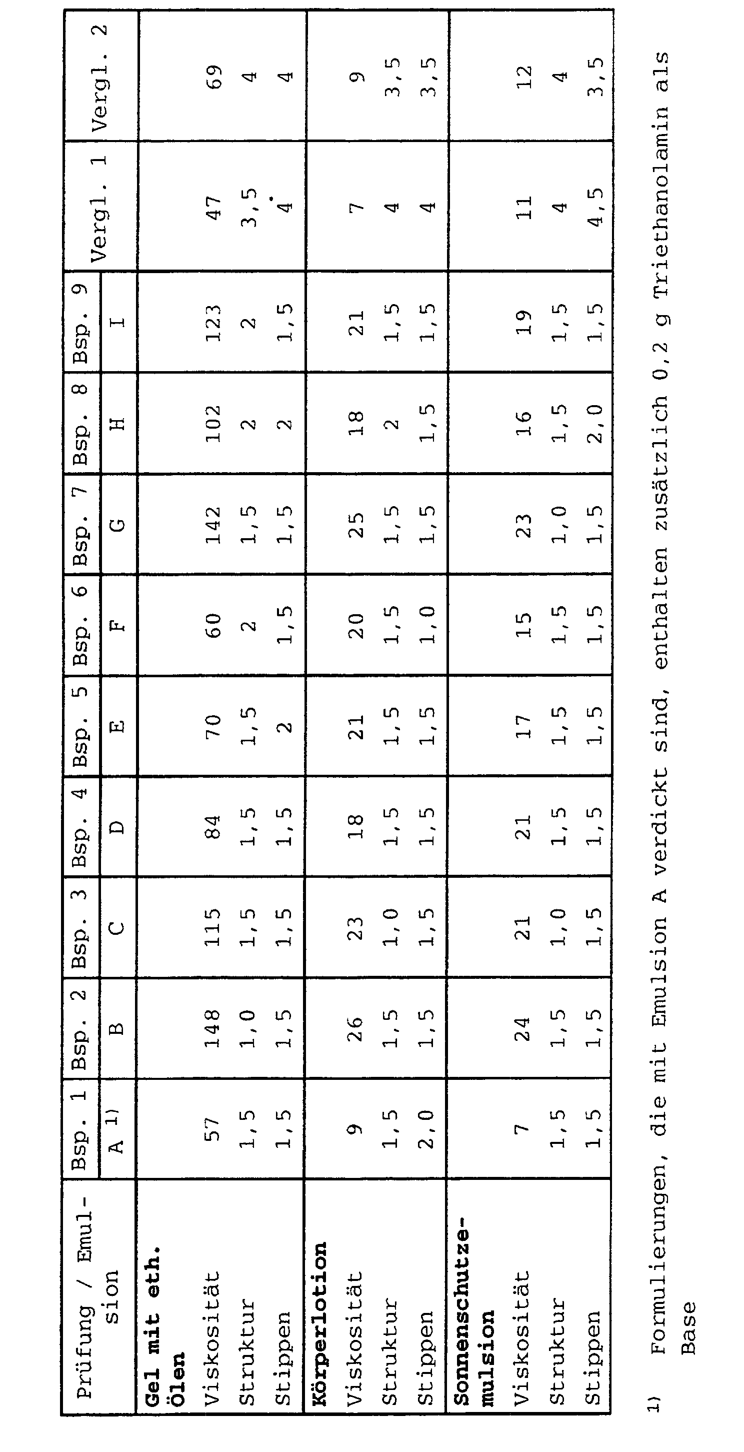

## Patentansprüche

1. Verwendung von W/O-Emulsionen, in denen vernetzte, wasserquellbare Polymerisate dispergiert sind,
wobei die Polymerisate bestehen aus
a) 35 - 100 Gew.-% ionischen Monomeren, wobei die ionischen Monomeren zu 5 - 80 % neutralisiert sind,
b) 0 - 65 Gew.-% nicht-ionischen Monomeren,
c) 0,3 - 1 Mol-%, bezogen auf a) und b) mindestens eines mindestens bifunktionellen Monomeren, und
wobei die Ölphase aus einem oder mehreren Fettsäureestern besteht,
als Verdicker in kosmetischen oder pharmazeutischen Zubereitungen.

2. Verwendung nach Anspruch 1, **dadurch gekennzeichnet, daß** die Ölphase aus Polyglycerinfettsäureestern besteht.

3. Verwendung nach Anspruch 1, **dadurch gekennzeichnet, daß** als ionische Monomere ungesättigte C₃-C₅-Carbonsäuren eingesetzt werden.

4. Verwendung nach Anspruch 1, **dadurch gekennzeichnet, daß** die W/O-Emulsion 0,25 bis 7 Gew.-%, bevorzugt 0,5 bis 5 Gew.-% eines Emulgators enthält.

5. Verwendung nach Anspruch 1, **dadurch gekennzeichnet, daß** die W/O-Emulsionen als Verdicker in haut- oder haarkosmetischen Zubereitungen eingesetzt werden.

6. W/O-Emulsionen, in denen vernetzte, wasserquellbare Polymerisate dispergiert sind,
wobei die Polymerisate bestehen aus
a) 35 - 100 Gew.-% ionischen Monomeren, wobei die ionischen Monomeren zu 5 - 80 % neutralisiert sind,
b) 0 - 65 Gew.-% nicht-ionischen Monomeren,
c) 0,3 - 1 Mol-%, bezogen auf a) und b) mindestens eines mindestens bifunktionellen Monomeren, und
wobei die Ölphase aus einem oder mehreren Polyglycerinfettsäureestern aus einer Polyglycerinmischung, die Diglycerin und Triglycerin enthält, mit Fettsäuregemischen, die Caprylund/oder Caprinsäure enthalten, besteht,

7. W/O-Emulsion nach Anspruch 6, **dadurch gekennzeichnet, daß** das ionische Monomere eine C₃-C₅-Carbonsäure ist.

8. W/O-Emulsion nach Anspruch 6, **dadurch gekennzeichnet, daß** das ionische Monomere Acrylsäure ist.

9. W/O-Emulsion nach Anspruch 6, **dadurch gekennzeichnet, daß** sie 0,25 bis 7 Gew.-%, bevorzugt 0,5 bis 5 Gew.-% eines Emulgators enthält.

10. Kosmetische oder pharmazeutische Zubereitungen, die eine W/O-Emulsion enthalten, in der vernetzte, wasserquellbare Polymerisate dispergiert sind, wobei die Polymerisate bestehen aus
a) 35 - 100 Gew.-% ionischen Monomeren, wobei die ionischen Monomeren zu 5 - 80 % neutralisiert sind,
b) 0 - 65 Gew.-% nicht-ionischen Monomeren,
c) 0,3 - 1 Mol-%, bezogen auf a) und b) mindestens eines mindestens bifunktionellen Monomeren, und
wobei die Ölphase aus einem oder mehreren Fettsäureestern besteht.

11. Zubereitungen nach Anspruch 10, **dadurch gekennzeichnet, daß** der oder die Fettsäureester Polyglycerinfettsäureester sind.

12. Zubereitung nach Anspruch 11, **dadurch gekennzeichnet, daß** die Polyglycerinfettsäureester Ester einer Polyglycerinmischung, die Diglycerin und Triglycerin enthält, mit Fettsäuregemischen, die Capryl- und Caprinsäure enthalten, sind.

## Claims

1. The use of W/O emulsions comprising dispersed therein crosslinked, water-swellable polymers consisting of
a) 35 - 100% by weight of ionic monomers, where 5 - 80% of the ionic monomers are in neutralized form,
b) 0 - 65% by weight of nonionic monomers,
c) 0.3 - 1 mol-%, based on a) and b), of at least one at least bifunctional monomer,
where the oil phase consists of one or more fatty acid esters,
as thickeners in cosmetic or pharmaceutical formulations.

2. The use as claimed in claim 1, wherein the oil phase consists of polyglycerol fatty acid esters.

3. The use as claimed in claim 1, wherein the ionic monomers employed are unsaturated C₃-C₅ carboxylic acids.

4. The use as claimed in claim 1, wherein the W/O emulsion comprises from 0.25 to 7% by weight, preferably from 0.5 to 5% by weight of an emulsifier.

5. The use as claimed in claim 1, wherein the W/O emulsions are employed as thickeners in cosmetic formulations for skin or hair.

6. A W/O emulsion comprising dispersed therein crosslinked, water-swellable polymers consisting of
a) 35 - 100% by weight of ionic monomers, where 5 - 80% of the ionic monomers are in neutralized form,
b) 0 - 65% by weight of nonionic monomers,
c) 0.3 - 1 mol-%, based on a) and b), of at least one at least bifunctional monomer, and
where the oil phase consists of one or more polyglycerol fatty acid esters of a polyglycerol mixture comprising diglycerol and triglycerol with fatty acid mixtures comprising caprylic and/or capric acid.

7. A W/O emulsion as claimed in claim 6, wherein the ionic monomer is a C₃-C₅ carboxylic acid.

8. A W/O emulsion as claimed in claim 6, wherein the ionic monomer is acrylic acid.

9. A W/O emulsion as claimed in claim 6, which comprises from 0.25 to 7% by weight, preferably from 0.5 to 5% by weight, of an emulsifier.

10. A cosmetic or pharmaceutical formulation which comprises a W/O emulsion comprising dispersed therein crosslinked, water-swellable polymers consisting of
a) 35 - 100% by weight of ionic monomers, where 5 - 80% of the ionic monomers are in neutralized form,
b) 0 - 65% by weight of nonionic monomers,
c) 0.3 - 1 mol-%, based on a) and b), of at least one at least bifunctional monomer, and
where the oil phase consists of one or more fatty acid esters.

11. A formulation as claimed in claim 10, wherein the fatty acid ester(s) is (are) polyglycerol fatty acid ester(s).

12. A formulation as claimed in claim 11, wherein the polyglycerol fatty acid esters are esters of a polyglycerol mixture comprising diglycerol and triglycerol with fatty acid mixtures comprising caprylic and capric acid.

## Revendications

1. Utilisation d'émulsions eau-dans-huile dans lesquelles des polymères réticulés, susceptibles de gonfler dans l'eau, sont dispersés,
les polymères étant constitués
a) de 35 à 100% en poids de monomères ioniques, les monomères ioniques étant neutralisés à 5-80%,
b) de 0 à 65% en poids de monomères non ioniques,
c) de 0,3 à 1% molaire, par rapport à a) et b), d'au moins un monomère au moins bifonctionnel,
la phase huileuse étant constituée d'un ou plusieurs esters d'acide gras,
comme épaississants dans des préparations cosmétiques ou pharmaceutiques.

2. Utilisation suivant la revendication 1, **caractérisée en ce que** la phase huileuse est constituée d'esters d'acide gras de polyglycérine.

3. Utilisation suivant la revendication 1, **caractérisée en ce que**, comme monomères ioniques, on met en oeuvre des acides carboxyliques en C₃-C₅ insaturés.

4. Utilisation suivant la revendication 1, **caractérisée en ce que** l'émulsion eau-dans-huile contient 0,25 à 7% en poids, de préférence 0,5 à 5% en poids, d'un agent émulsionnant.

5. Utilisation suivant la revendication 1, **caractérisée en ce que** les émulsions eau-dans-huile sont mises en oeuvre comme épaississants dans des préparations cosmétiques pour la peau ou les cheveux.

6. Emulsions eau-dans-huile, dans lesquelles des polymères réticulés, susceptibles de gonfler dans l'eau, sont dispersés,
les polymères étant constitués
a) de 35 à 100% en poids de monomères ioniques, les monomères ioniques étant neutralisés à 5-80%,
b) de 0 à 65% en poids de monomères non ioniques,
c) de 0,3 à 1% molaire, par rapport à a) et b), d'au moins un monomère au moins bifonctionnel,
la phase huileuse étant constituée d'un ou plusieurs esters d'acide gras de polyglycérine à base d'un mélange de polyglycérine, qui contient de la diglycérine et de la triglycérine, avec des mélanges d'acides gras, qui contiennent de l'acide caprylique et/ou de l'acide caprique.

7. Emulsion eau-dans-huile suivant la revendication 6, **caractérisée en ce que** le monomère ionique est un acide carboxylique en C₃-C₅.

8. Emulsion eau-dans-huile suivant la revendication 6, **caractérisée en ce que** le monomère ionique est de l'acide acrylique.

9. Emulsion eau-dans-huile suivant la revendication 6, **caractérisée en ce qu'**elle contient 0,25 à 7% en poids, de préférence 0,5 à 5% en poids, d'un agent émulsionnant.

10. Préparations cosmétiques ou pharmaceutiques, qui contiennent une émulsion eau-dans-huile dans laquelle des polymères réticulés, susceptibles de gonfler dans l'eau, sont dispersés,
les polymères étant constitués
a) de 35 à 100% en poids de monomères ioniques, les monomères ioniques étant neutralisés à 5-80%,
b) de 0 à 65% en poids de monomères non ioniques,
c) de 0,3 à 1% molaire, par rapport à a) et b), d'au moins un monomère au moins bifonctionnel,
la phase huileuse étant constituée d'un ou plusieurs esters d'acide gras.

11. Préparations suivant la revendication 10, **caractérisées en ce que** le ou les esters d'acide gras sont des esters d'acide gras de polyglycérine.

12. Préparations suivant la revendication 11, **caractérisées en ce que** les esters d'acide gras de polyglycérine sont des esters d'un mélange de polyglycérine, qui contient de la diglycérine et de la triglycérine, avec des mélanges d'acides gras, qui contiennent de l'acide caprylique et de l'acide caprique.
